# EUROPEAN PATENT SPECIFICATION

(11) **EP 1 487 507 B1**
(45) Date of publication and mention of the grant of the patent: **12.07.2006**
(21) Application number: 03708358.1
(22) Date of filing: 24.03.2003
(51) Int. Cl.: A61L 15/28, A61L 15/42

(54) **WOUND DRESSING MATERIALS COMPRISING CHEMICALLY MODIFIED POLYSACCHARIDES**
WUNDVERBAND ENHALTEND CHEMISCH MODIFIZIERTE POLYSACCHARIDE
MATERIAUX DE PANSEMENT COMPRENANT DES POLYSACCHARIDES MODIFIES CHIMIQUEMENT

(30) Priority: 25.03.2002 GB 0207006
(43) Date of publication of application: 22.12.2004
(73) Proprietor: Johnson & Johnson Medical Ltd., Edinburgh EH2 4NH (GB)
(72) Inventor: SCHMIDT, Ryszard, Jan, Barnoldswick BB18 6HJ (GB)
(74) Representative: James, Anthony Christopher W.P.
(86) International application number: PCT/GB2003/001206
(87) International publication number: WO 2003/080134

(56) References cited:
- EP-A- 0 640 622
- EP-A- 0 718 312
- EP-A- 0 893 451
- WO-A-94/04129
- WO-A-94/13333
- US-A- 2 661 349
- US-A- 5 770 711
- CONSTANTIA E. K. ET AL: "Thiolated polymers - thiomers: development and in vitro evaluation of chitosan-thioglycolic acid conjugates" BIOMATERIALS, vol. 22, 2001, pages 2345-2352, XP002242794

## Description

The present invention relates to wound dressings and other therapeutic compositions comprising certain chemically modified polysaccharides, processes suitable for the preparation of such compositions, and to the use of such compositions as wound dressings.

Concentrations of reactive oxygen species such as hydroxyl radicals (·OH), singlet oxygen (¹O₂), hydroperoxyl radicals (·OOH), superoxide radical anions (·O₂⁻), and hydrogen peroxide (H₂O₂) can rise in damaged tissues, producing a condition known as oxidative stress. The presence of a low level of reactive oxygen species may be advantageous in the early stages of wound healing by both attracting and activating macrophages which engulf and kill bacteria and release cytokines and growth factors. However, prolonged and more severe oxidative stress may delay healing because it will produce chronic inflammation, divert available energy supply towards antioxidant defence at the expense of tissue reconstruction, and increase levels of matrix metalloproteinases which cause tissue breakdown. In more severe cases, elevated levels of reactive oxygen species can give rise to hydrogen peroxide-induced senescence or apoptosis (that is, programmed cell death) or tissue necrosis (that is, uncontrolled cell death and therefore permanent tissue damage).

Under mild oxidative stress, it is thought that hydrogen peroxide (H₂O₂) is the dominant species present, being formed rapidly from superoxide by the enzyme superoxide dismutase. This enzyme-mediated dismutation reaction also minimises the production of singlet oxygen that can arise when superoxide is produced too rapidly and therefore has the opportunity to dismutate spontaneously without enzyme assistance. Rapid enzyme-mediated dismutation of superoxide also minimises levels of hydroperoxyl radical, the unionised form of superoxide. Levels of hydrogen peroxide are in turn kept low by the actions of catalase and glutathione peroxidase. Thus, under mild oxidative stress conditions when hydrogen peroxide levels are slightly raised (around 10⁻⁸ to 10⁻⁴ molar), it has been found that the rate of cell proliferation in fibroblast cultures is stimulated.

Accordingly, the healing of chronic wounds may be assisted by the use of antioxidant wound dressings that react specifically with excess reactive oxygen species such as those listed above and hence reduce the level of oxidative stress.

US-A-5667501 describes compositions comprising chemically modified polymers grafted with chemical groups that confer antioxidant activity as measured by a diphenylpicrylhydrazyl (DPPH) test and that also generate low levels of hydrogen peroxide by reaction with molecular oxygen in the wound bed to stimulate macrophage activity and fibroblast proliferation. The compositions may be used to promote the healing of chronic wounds. Preferably, the polymer is a polymer bearing hydroxyl, carbonyl or amide functional groups, or a polysaccharide bearing hydroxyl functional groups, said functional groups having been converted to derivatives that are persistent free radicals or precursors of persistent free radicals, that is to say they are free radical scavenging antioxidant groups.

US-A-5612321 describes compositions comprising polysaccharides grafted with antioxidants on at least one hydroxyl group of the polysaccharide. The compositions may be used inter alia to promote the healing of chronic wounds. Preferably, the polysaccharide is hyaluronic acid and the antioxidant group comprises a phenol group.

In the complex biochemistry of infected and/or chronic wounds, it is also thought that iron plays an important role. For example, iron may play a role in catalysing the generation of the above-described reactive oxygen species, and thereby may contribute to wound chronicity. Furthermore, bacteria require iron for metabolism and can secrete siderophores for the purpose of scavenging iron. It therefore appears that removal of free iron from the wound fluid, preferably without removal of other dissolved species such as zinc that promote wound healing, could assist in reducing both wound colonisation or infection by bacteria and wound chronicity.

US-A-6156334 describes novel wound coverings comprising chelators such as the siderophore deferoxamine grafted onto a suitable carrier through a linker molecule, which remove interfering factors such as iron from the wound bed. Foitzik A *et al.* in the Proceedings of the Joint Meeting of the European Tissue Repair Society/The Wound Healing Society, Bordeaux, 24th - 28th August 1999, describe treatment of chronic venous ulcers by means of a gauze dressing having deferoxamine coupled thereto. The resulting iron-absorbing dressing is expensive to manufacture, and its antioxidant properties and affinity for beneficial species such as zinc are unknown.

GB-A-2067203 describes polysaccharides that have been modified by grafting ethylenediamine tetra-acetate (EDTA) units onto the polysaccharide chain. The resulting modified polysaccharides complex strongly with dissolved metal ions. They are indicated for use in the recovery of heavy metals from solution, and separation of metals in analytical chemistry. There is no disclosure of any medical use of the modified polysaccharides.

EP-A-0718312 describes acidic polysaccharides that have been cross-linked by reaction in solution in a polar, aprotic solvent with di- or polyanhydrides. A preferred example is hyaluronic acid cross-linked with disahydrides. The resulting cross-linked polysaccharides may be used for a range of medical applications, including as wound dressings for chronic wounds and burns.

EP-A-0640622 describes drug delivery systems, wherein a drug molecule is bound to a polysaccharide carrier through a linker group comprising an oligopeptide, wherein the linker group optionally also comprises a polybasic acid moiety. There is no teaching or suggestion that the resulting molecules have antioxidant effects, or that they may be useful in the treatment of wounds.

WO94/04129 describes various thiol-containing small molecules for use as antioxidant additives in cosmetic sunscreen compositions.

US-A-5789507 describes water absorbent resins produced by radical polymerisation of a water soluble monomer, such as a vinyl sulfonate or an acrylate, in the presence of a polysaccharide and a cross-linking agent. Presumably this results in cross-linking between the polysaccharide and the polyacrylate. The resulting superabsorbent polymers may be used in a range of absorbent products, including as the absorbent layer in bandages.

US-A-4997935 describes modified cellulose materials for the manufacture of biocompatible dialysis membranes. One of the substituent groups present on the cellulose may be -CH(COOH)₂. There is no suggestion in the reference of the any therapeutic compositions comprising the modified cellulose materials.

US-A-4152170 describes cross-linked pullulans that have been produced by reacting pullulan with cyclic anhydride cross-linking agents. The resulting materials may be used as superabsorbents in sanitary and medical applications. There is no disclosure of a therapeutic composition containing the cross-linked pullulans.

The present invention provides therapeutic composition comprising a chemically modified polysaccharide, wherein at least one saccharide residue has grafted thereto a side chain bearing two or more carboxylate groups, wherein the side chain has only one linkage to the polysaccharide, and wherein the modified polysaccharide is hydrophilic but has a solubility of less than about 1 gram per litre in water at 25°C.

The therapeutic composition preferably comprises at least 1wt% on a dry basis of the chemically modified polysaccharide, more preferably at least 5wt.%, still more preferably at least 10wt.%, 25wt% or 50wt%. In certain embodiments, the composition consists essentially of the chemically modified polysaccharide.

The side chain on the modified polysaccharide is a branch group having only one linkage to a polysaccharide molecule. That is to say, it is not a cross-linking group. It has been found that the presence of two carboxylate groups provides a side chain that is a surprisingly effective and selective ligand for the removal of iron from solution. Furthermore, it has been found that the presence of the side chain with two carboxylate groups confers an unexpected reactivity of the polysaccharide with free radicals that could render it useful for the removal of reactive oxygen species from the wound environment.

The two carboxylate groups may be substituted geminally on the side chain, or preferably vicinally or more distantly on said side chain. That is to say, the two carboxylate groups may be attached to the same carbon atom, or more preferably to adjacent or more distantly separated carbon atoms. In any case, the two carboxylate groups are preferably configured and arranged so that both of the carboxylate groups can form a complex with a single metal ion, such as a ferric ion. That is to say, the side chain preferably can form a chelate (bidentate or multidentate) complex with the ion through the carboxylate groups. This multidentate complexation results in much more effective removal of the metal ions from wound fluid.

Preferably, at least one of the carboxylate groups is attached to an unsaturated carbon atom, such as in an alkylene or alkenylene chain, since such attachment can fix the carboxylate groups in a configuration that is especially suitable for complexation with iron. Furthermore, the conjugation of the carboxylate group to the unsaturated carbon assists the free radical scavenging property of the carboxylate group by stabilizing the carboxylate radical.

More than two carboxylate groups may be present on the side chain. The terms "-COOH" or "carboxylate group" herein refers not only to the group -COOH itself, but also to all pharmaceutically acceptable salts, ion pairs, or protected carboxyl groups, for example readily hydrolysable anhydrides, lactones or esters thereof.
Preferably, the side chain bearing the carboxylate groups is linked to the polysaccharide chain through an ester or amide linkage. Preferably, the linkage is made through an -OH group or -NH₂ group on the polysaccharide, though esterification or amidation of carboxylate groups on polysaccharides such as alginates could also be envisaged within the scope of the invention.

Preferably, the side chain bearing the carboxylate groups is a straight or branched or cyclic group comprising from 1 to 10 alkyl or alkylene carbon atoms, optionally interrupted by -O, -S-, -CO- or -NH-, preferably containing at least one C=C double bond, and optionally further substituted with one or more substituents selected from the group -OH, -NH₂, and halogen. Such side groups can conveniently be formed by reaction of the polysaccharide with a carboxyl-substituted cyclic anhydride, as described in more detail below.

Preferably, the side group comprises 2 to 4 alkyl or alkylene carbon atoms and one C=C double bond. More preferably, the modified polysaccharide comprises two carboxylate groups attached to a C₃ alkenyl chain, in particular:

-O-CO-CH=C(COOH)CH₂COOH

Such side chains can be formed by reacting a polysaccharide with cis-aconitic anhydride as described further below.

The most preferred substituents are those that provide higher affinity for iron, than for zinc, in combination with DPPH free radical scavenging activity as determined by the assays capable of measuring these activities that are described in detail below.

In certain embodiments, the polysaccharide is further chemically modified by the introduction of one or more thiol groups. It has also been found that the presence of thiol groups confers a surprising high affinity for iron with a low affinity for zinc and additionally a reactivity with free radicals that could render the derivatised polysaccharide useful for the removal of reactive oxygen species from the wound environment.

The thiol groups may replace hydroxyl groups of the polysaccharide, but preferably the thiol groups are present on a side chain that is grafted onto the polysaccharide through an ester or ether linkage. Preferably, the side chain has from 1 to 8 carbon atoms. A preferred side chain is 2-ethoxythiol, for example in a modified polysaccharide comprising a moiety of the following general formula.

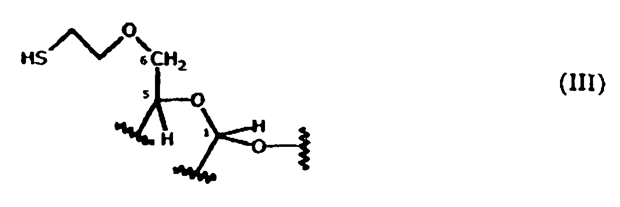

Such side chains of structure (III) can conveniently be formed by reacting a polysaccharide or a modified polysaccharide, for example hydroxyethyl cellulose, with Lawesson's reagent as described further below.

Preferably, the chemically modified polysaccharide according to the present invention is a modified or derivatised glycan or aminoglycan.

Preferably, the modified or derivatised glycan is a modified or derivatised guluronomannuronan such as an alginate or alginate derivative.

Preferably, the modified or derivatised glycan is a modified or derivatised glucan such as cellulose or a cellulose derivative.

More preferably, the modified or derivatised glycan is a hydroxyalkyl cellulose for example hydroxyethyl cellulose, and the modified or derivatised aminoglycan is a modified or derivatised chitin/chitosan.

Optionally, the modified polysaccharide may comprise a mixture of two or more modified glycans, aminoglycans or derivatives thereof.
Each polysaccharide molecule will preferably comprise a plurality of the side chains comprising two or more carboxylate groups. Preferably, at least about 1% of the saccharide residues in the modified polysaccharide according to the present invention are modified by the grafting of side chains in accordance with the invention, that is to say the side chains having at least two carboxylate groups. More preferably, at least about 5% of the saccharide residues are so modified, and still more preferably at least about 10% to about 20% of the saccharide residues are so modified. In certain embodiments, the modification of the polysaccharide is primarily a surface modification. That is to say, the concentration of the side chains is higher at the surface of the polysaccharide than in the bulk of the polysaccharide. This is the case, for example, if the modification has been carried out by reaction of the polysaccharide with the modifying reagent in a solvent in which the polysaccharide is insoluble (see below).

Preferably, the modified polysaccharide according to the present invention has a free radical activity, that is to say an antioxidant activity, of at least about 15% in the diphenylpicrylhydrazyl (DPPH) test, measured as percentage reduction in absorbance at 524nm after 4 hours of a 0.5%w/v dispersion of the polysaccharide in 10⁻⁴ M DPPH, as described further hereinbelow in Procedure 2. Preferably the percentage reduction in absorbance in the DPPH test is at least about 25%, more preferably at least about 50%, and most preferably at least about 75%.

Preferably, the modified polysaccharide according to the present invention will absorb water or wound fluid and hence become wet, swell or become a gelatinous mass but will not spontaneously dissolve or disperse therein. That is to say, it is hydrophilic but has a solubility of preferably less than about 1g/liter in water at 25°C. Low solubility renders such polysaccharides especially suitable for use as wound dressings to remove iron and reactive oxygen species from the wound fluid.

The modified polysaccharide according to the present invention preferably forms complexes with iron selectively over zinc. That is to say, the modified polysaccharide preferably sequesters a higher fraction of the iron ions than zinc ions when treated with a serum containing both types of ions as described in detail in Procedure 1.

More preferably, the percentage of Fe³⁺ ions removed is at least about twice the percentage of the Zn²⁺ ions removed, more preferably it is at least about four times the percentage of the Zn²⁺ ions removed. Preferably, a complex of the polysaccharide with Fe³⁺ in water at neutral pH has a stability constant of at least 10³, preferably at least 10⁶.

The therapeutic compositions according to the present invention may be provided in the form of beads, flakes, powder, and preferably in the form of a film, a fibrous pad, a web, a woven or non-woven fabric, a freeze-dried sponge, a foam or combinations thereof. Such formats are especially useful for wound dressings, which can easily be made by derivatisation of or by grafting of side chains onto polysaccharides already provided in the relevant formats. In addition to the solid formats discussed above, the compositions may also be in the form of a gel or viscous fluid for topical application.

Preferably, the therapeutic compositions according to the present invention are sterile. Preferably, the therapeutic compositions according to the present invention are packaged in a microorganism-impermeable container.

The antioxidant and iron sequestering properties of the modified polysaccharides in the therapeutic compositions according to the present invention suggest applications in a range of medical applications, including the treatment of acute surgical and traumatic wounds, burns, fistulas, venous ulcers, arterial ulcers, pressure sores (otherwise known as decubitus ulcers), diabetic ulcers, ulcers of mixed aetiology, and other chronic or necrotic wounds and inflammatory lesions and disorders. Another therapeutic application that is likely to be useful is in the treatment or prevention of lipodermatosclerosis (LDS). LDS is a pre-ulcerous discoloring and hardening of the skin in patients suffering from venous insufficiency leading ultimately to varicose ulcers. It is thought that excess iron plays a part in the mechanism of LDS, and therefore that the iron sequestering properties of these modified polysaccharides will be useful in the treatment and prevention of LOS.

Accordingly, in preferred embodiments of the present invention the therapeutic composition is a wound dressing composition.

The present invention further provides the use of a therapeutic composition according to the present invention for the preparation of a medicament for the treatment of a wound. Preferably, the wound is a chronic wound. More preferably, the chronic wound is selected from the group consisting of ulcers of venous, arterial or mixed aetiology, decubitus ulcers, or diabetic ulcers.

The present invention further provides the use of a therapeutic composition according to the present invention for the preparation of a medicament for the treatment or prevention of lipodermatosclerosis.

In a further aspect, the present invention provides a method of treatment of a wound in a mammal comprising applying thereto a therapeutically effective amount of a therapeutic composition according to the present invention. Preferably, the wound is a chronic wound.

In a further aspect, the present invention provides a method of treatment or prevention of lipodennatosclerosis in a mammal comprising applying to LDS skin, or to skin liable to develop LDS, a therapeutically effective amount of a modified polysaccharide according to the present invention. Skin liable to develop LDS would include, for example, the lower leg of individuals suffering from venous insufficiency or varicose veins.

The present invention further provides a process for the production of a modified polysaccharide comprising the step of reacting a polysaccharide with a cyclic anhydride, wherein the cyclic anhydride is substituted with at least one carboxylate group. Preferably, the cyclic anhydride has general structure (IV):

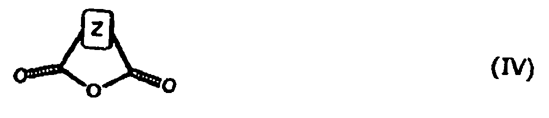

Wherein Z is a straight or branched or cyclic group comprising from 1 to 10 alkyl or alkylene carbon atoms, optionally interrupted by -O-, -S-, -CO- or -NH-, preferably containing at least one C=C double bond, and optionally further substituted with one or more substituents selected from the group -OH, -NH₂, and halogen, and wherein Z is substituted with at least one carboxylate group.

Preferably Z comprises 2 to 4 alkyl or alkylene carbon atoms and more preferably it contains at least one C=C double bond.

Preferably, the cyclic anhydride of general structure (IV) is cis-aconitic anhydride. That is to say, Z is -CH=C(COOH)-.

Preferably, the cyclic anhydride of general structure (IV) is reacted with said polysaccharide in an amount of at least 0.1 moles per mole of saccharide residues of the polysaccharide.

Preferably, the reaction with the cyclic anhydride is carried out in an aprotic solvent, such as toluene. In certain embodiments, the polysaccharide is not soluble in the solvent, whereby the reaction with the cyclic anhydride is primarily a surface modification of the polysaccharide.

In certain embodiments the process of the present invention further comprises the step of reacting the polysaccharide or polysaccharide derivative with Lawesson's reagent having the structure (V):

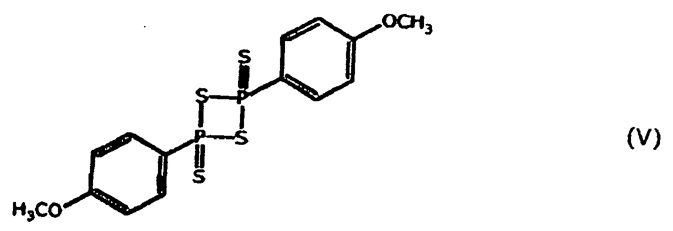

Preferably, the Lawesson's reagent is reacted with said polysaccharide or polysaccharide derivative in an amount of at least 0.1 moles per mole of saccharide residues of the polysaccharide or polysaccharide derivative.

The present invention further provides modified polysaccharides obtainable by processes according to the invention. Preferably, the modified polysaccharides have structures as hereinbefore defined for the first aspect of the invention.

Specific embodiments of the present invention will now be described further with reference to the drawings, in which:

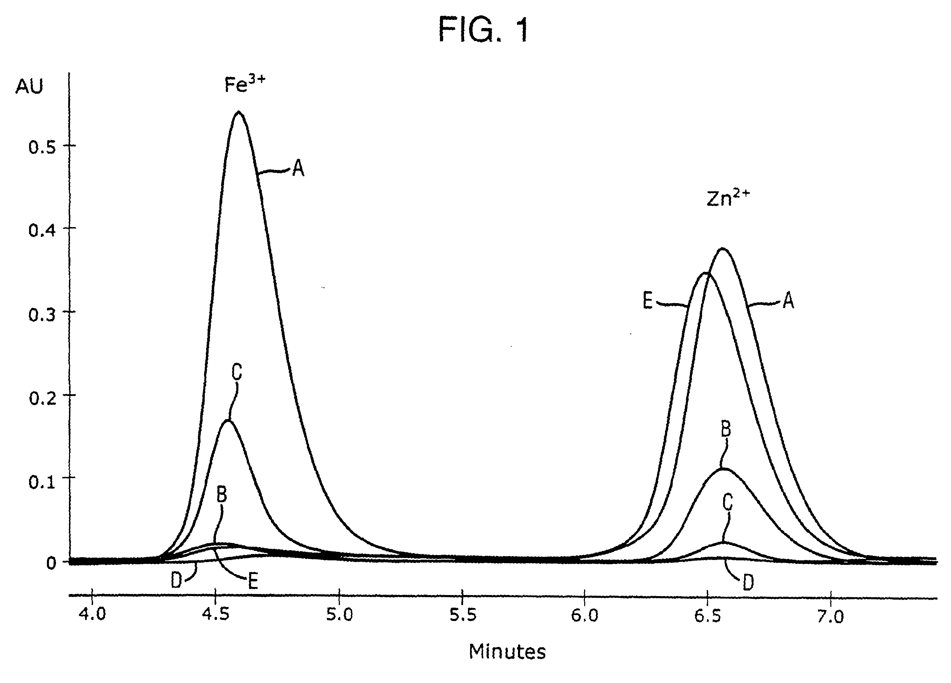

Figure 1 shows chromatograms of iron and zinc ion concentrations for a control serum (D) containing no added iron or zinc, a control serum containing added iron and zinc ions (A), the serum A after treatment with polysaccharides according to the present invention (B,C), and the serum A after treatment with a thiolated polysaccharide (E, reference example); and

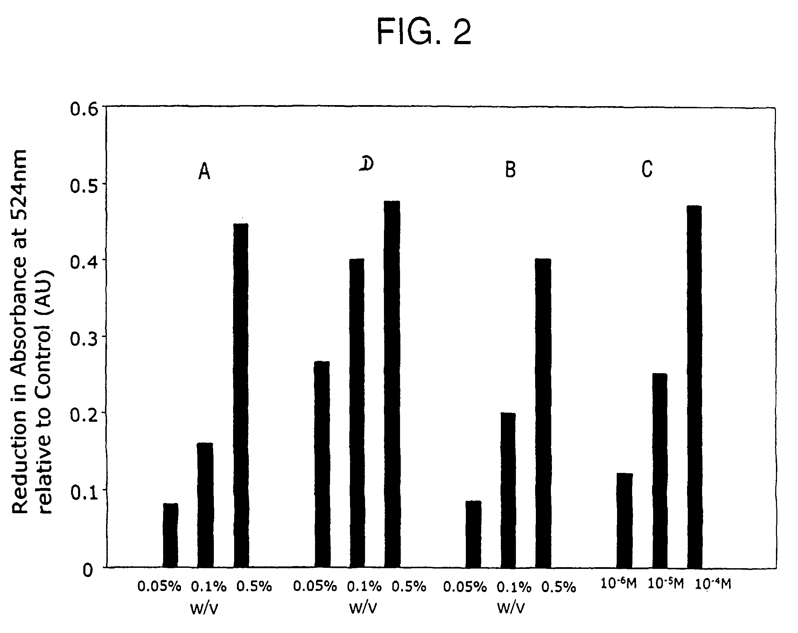

Figure 2 shows the absolute reduction in absorbance of DPPH at 524nm versus concentration of antioxidant for ascorbic acid (C, reference example), polysaccharides according to the present invention (A,B), and a thiolated polysaccharide (D, reference example). The absorbance of the starting DPPH solution was 0.506AU.

### Example 1

A modified chitin/chitosan was prepared by reaction with cis-aconitic anhydride, as follows.

Chitin/chitosan (1.73g, 10.0mmol based on saccharide residues) was placed in a dry 250ml three-neck round bottomed flask with 30ml of anhydrous toluene. The flask was fitted with a water condenser and thermometer and the mixture was then stirred and heated to 80°C under a nitrogen atmosphere. cis-Aconitic anhydride (Aldrich Chemical Co. catalog number 21,780-8) (4.68g, 30.0mmol) was added in portions over 10 minutes. The reaction temperature was maintained at 80°C for a further three hours. The mixture was allowed to cool to 30°C before deionised water (30ml) was added. The mixture was then stirred for 30 minutes to allow for complete hydrolysis of the unreacted anhydride, then centrifuged to separate the solid modified chitiivchitosan from dissolved components. The solid pellets from the centrifuge were placed in a beaker and 250ml of saturated aqueous sodium carbonate (Na₂CO₃) was added slowly. The mixture was stirred vigorously until a gelatinous solution had formed and all lumps had been removed. Aqueous 1M hydrochloric acid (HCl) was then added carefully until effervescence stopped. The modified chitosan was thereby precipitated, and the precipitate was allowed to stand overnight before again being centrifuged and further washed with water and ethanol prior to final centrifugation. Finally, the washed pellets were blast frozen and freeze dried overnight.

The mass of the product was 1.94g, representing an increase of 0.21 g over the starting chitin/chitosan. This suggests that at least 13.5% of the saccharide residues on the chitosan have been modified by conversion of -NH₂ into -NHCOCH₂C(CO₂H)=CHCO₂H.

The aconitylated chitin/chitosan exhibited both selective complexation with Fe³⁺ in preference to Zn²⁺ and antioxidant properties, as determined in procedures 1 and 2 described below. The results for this material are shown as graph B in Fig. I and as plot A in Fig. 2. Neither of these properties is observed for the unmodified chitin/chitosan starting material.

### Example 2

A sample of hydroxyethyl cellulose was modified by treatment with cis-aconitic anhydride as follows.

Hydroxyethyl cellulose (2.06g, 10.0mmols based on saccharide residues) was reacted with cis-aconitic anhydride (4.68g, 30.0mmols) as described above in Example 1. The mass of the product was 3.35g, suggesting that at least 0.83 -OH groups per saccharide residue had been modified to -OCOCH₂C(CO₂H)=CHCO₂H groups by the reaction.

The aconitylated hydroxyethyl cellulose exhibited an affinity for both Fe³⁺ and Zn²⁺ (but with different selectivity than the material of Example 1) and also antioxidant properties as determined in procedures 1 and 2 described below. The results for this material are shown as graph C in Fig. 1 and as plot B in Fig. 2. Neither of these properties is observed for the unmodified hydroxyethyl cellulose starting material.

### Example 3 (Reference Example)

The effect of thiol groups on the properties of polysaccharides was studied as follows.

A sample ot hydroxyethyl cellulose was modified by treatment with Lawesson's reagent as follows. Hydroxyethyl cellulose (10.3g, 50mmoles) was placed in a dry 250ml three-neck round bottomed flask with 150ml of anhydrous toluene. To this was added Lawesson's reagent (Aldrich Chemical Co. catalog number 22,743-9; 2.02g; 5mmoles). The flask was fitted with a water condenser and the mixture was then stirred and heated under reflux in a nitrogen atmosphere for 4 hours. The reaction mixture was then allowed to cool to 30°C before quenching with aqueous 1M potassium hydroxide (KOH) solution (100ml) to ensure complete hydrolysis of Lawesson's reagent. After standing for a further 15 minutes, liquid phases were decanted and collected for disposal. The remaining solid material was washed with aqueous 1M KOH (100ml x 3) and with aqueous 1M hydrochloric acid (100ml x 3), decanting the washings between each aliquot of wash solution. The remaining solid material was then washed with deionised water (100ml x 3) and with ethanol (100ml x 3). Centrifugation was necessary between washings. The resultant pellets were placed on a tray and blast frozen before freeze drying. Yield of product was 4.03g. The degree of thiolation could be increased by increasing the initial proportion of Lawesson's reagent to polysaccharide; loss of product during washing could be decreased by using isopropanol/water mixtures as solvents for the alkaline, acidic, and neutral washes.

The thiolated hydroxyethyl cellulose exhibited a high affinity for Fe^{3"1"} and a particularly low affinity for Zn²⁺ and also antioxidant properties as determined in procedures 1 and 2 described below. The results for this material are shown as graph E in Fig. 1 and as plot D in Fig. 2. Neither of these properties is observed for the unmodified hydroxyethyl cellulose starting material.

### Procedure 1

The ability of modified polysaccharides according to the present invention to sequester iron ions selectively over zinc ions in aqueous media was determined as follows.
The modified polysaccharide was added with stirring to a solution containing iron and/or zinc ions in a suitable matrix such as buffer, serum-containing buffer or cell culture medium that is intended to simulate wound fluid. Following a suitable incubation period, ion chromatography was used to determine the levels of uncompiexed Fe³⁺ and/or Zn²⁺ ions remaining in solution.

In the present example, solutions of FeCl₃.6H₂O and ZnSO₄.7H₂O in water were added to Dulbecco's Modified Eagle's Medium (DMEM; Sigma Chemical Co. catalog number D 5546) containing 10% v/v calf serum (Sigma Chemical Co. catalog number N 4637) to produce final concentrations of 50 ppm Fe³⁺ and 50 ppm Zn²⁺. The mixed solution was incubated at 37°C with gentle agitation for 16 hours with the modified polysaccharide present at a concentration of 2% w/v. The samples were then centrifuged, and a 0.9ml sample of the supernatant was analysed for iron and zinc content by the following method.

The centrifuged solution was treated with 0.1ml of a 20% w/v solution of trichloroacetic acid (TCA) to give a final concentration of 2% w/v TCA. The tube was vortexed for 10 seconds and then centrifuged for 15 minutes or longer to remove solids. The supernatant (0.5ml) was added to a clean dry HPLC vial to which was added 0.5ml of 0.5M nitric acid (HNO₃) prepared in deionised water.

The samples were then analysed for free iron and zinc ions using a Dionex DX500 HPLC apparatus according to the following method details:

| | |
|---|---|
| Instrument: | Dionex DX500 HPLC |
| Column: | Ionpac CS5A Analytical Column (Dionex part no. 046100) |
| | lonpac CS5A Guard Column (Dionex part no. 046104) |
| Eluants: | A - Deionised water |
| | B - Metpac PDCA reagent concentrate (part no. 046088) |
| | C - Metpac PAR reagent diluent (Dionex part no. 046094) |

| | |
|---|---|
| Postcolumn | |
| Reagent: | 4-(2-pyridylazo) resorcinol monosodium salt (PAR) 0.12g/l |
| | Detector |
| Wavelength: | 530nm |
| Temperature: | 25°C |
| Flow rate: | 1.2ml.min (80% eluant A: 20% eluant B) in column; |
| | 0.6ml/min eluant C postcolumn |
| Sample size: | 100 µl |

Data for the samples prepared in accordance with Examples 1-3 are shown in Figure 1. It can be seen that the modified polysaccharides remove Fe³⁺ and Zn²⁺ ions from Dulbecco's Modified Eagle's Medium containing calf serum with varying selectivities.

### Procedure 2

The ability of the modified polysaccharide materials to react with and remove oxygen containing free radicals is assessed by the DPPH test described in WO94/13333, the entire content of which is expressly incorporated herein by reference. The test is adapted from that described by Blois M.S. in Nature 181: 1199 (1958), and Banda P.W. *et al.,* in Analytical Letters 7: 41 (1974).

Briefly, modified polysaccharide under test (2.5mg; 5mg; & 25mg sample sizes) was suspended in 2.5ml of 0.1M pH 7.0 phosphate buffer. A solution of diphenylpicrylhydrazyl (DPPH) in methanol (10⁻⁴ M) was added in an amount of 2.5 ml and the mixture was shaken and stored in the dark at 20°C. The samples were assessed by measurement of their light absorbance at 524nm over 6 hours in comparison with a control, particular attention being paid to the figure after 4 hours. The percentage reduction of absorbance relative to the control after 4 hours gives the DPPH test value, with a reproducibility generally of ±5%. This value may conveniently be expressed in terms of a simple reduction in absorbance units (AU) relative to the control as shown in Figure 2 in which the DPPH control solution containing no test sample produced an absorbance reading of 0.506 AU. Ascorbic acid, a well known antioxidant, provides a useful positive control substance for comparative purposes.

Application of this test to the products of Examples 1-3 resulted in DPPH test values of 80-90% for the positive control (10⁻⁴M) and for all three materials at their highest concentration (0.5% w/v) after 4 hours, as shown in Fig. 2. In contrast, the unmodified polysaccharides chitin/chitosan and hydroxyethyl cellulose exhibited much lower DPPH values of less than 15%.

The above embodiments have been described by way of example only. Many other embodiments falling within the scope of the accompanying claims will be apparent to the skilled reader.

## Claims

1. A therapeutic composition comprising a chemically modified polysaccharide, wherein at least one saccharide residue has grafted thereto a side chain comprising two or more carboxylate groups wherein the side chain has only one linkage to the polysaccharide, and wherein the modified polysaccharide is hydrophilic but has a solubility of less than about 1 gram per litre in water at 25°C.

2. A therapeutic composition according to claim 1, wherein one or more hydroxyl functions on the saccharide units or on the side chain have been converted into a thiol function.

3. A therapeutic composition according to claim 1 or 2, wherein the two carboxylate groups are substituted 1,1 (geminally), 1,2 (vicinally) or 1,3 on said side chain.

4. A therapeutic composition according to claim 2, wherein one or more hydroxyl groups on the saccharide units have been converted into thiol groups.

5. A therapeutic composition according to claim 2, wherein one or more hydroxyl groups on side chains grafted onto the saccharide units have been converted into thiol groups.

6. A therapeutic composition according to any preceding claim wherein the side chain is linked to the polysaccharide chain by an ester or amide linkage.

7. A therapeutic composition according to any preceding claim, wherein the side chain bearing the carboxylate groups is a straight or branched or cyclic group comprising from 1 to 10 alkyl or alkylene carbon atoms, optionally interrupted by -O-, -S-, -CO- or -NH-, optionally containing at least one C=C double bond, and optionally further substituted with one or more substituents selected from the group -OH, -NH₂, and halogen.

8. A therapeutic composition according to any preceding claim, wherein at least one of the said carboxylate groups is bonded to an unsaturated carbon atom on said side chain.

9. A therapeutic composition according to any preceding claim, wherein the modified polysaccharide comprises a side chain of general structure -X-COCH=C(COOH)CH₂COOH, wherein X is O or NH.

10. A therapeutic composition according to any preceding claim, wherein the modified polysaccharide is modified chitin/chitosan or a modified cellulose or a modified hydroxyalkyl cellulose or a modified alginate.

11. A therapeutic composition according to any preceding claim, wherein at least about 1% of the saccharide residues of said modified polysaccharide are modified.

12. A therapeutic composition according to claim 11, wherein at least about 5% of the saccharide residues are modified.

13. A therapeutic composition according to any preceding claim, wherein the modified polysaccharide has a free radical activity in the diphenylpicrylhydrazyl (DPPH) test for antioxidant activity as herein defined of at least about 15%.

14. A therapeutic composition according to any preceding claim that is substantially insoluble in water.

15. A therapeutic composition according to any preceding claim, wherein the modified polysaccharide complexes with Fe³⁺ ions selectively over Zn²⁺ ions.

16. A therapeutic composition according to any preceding claim, wherein the modified polysaccharide is predominantly surface modified.

17. A therapeutic composition according to any preceding claim, wherein the carboxylate groups are carboxyl groups.

18. A therapeutic composition according to any preceding claim in the form of beads, flakes, powder, a film, a fibrous pad, a web, a woven or non-woven fabric, a freeze-dried sponge, a foam or any combination thereof.

19. A therapeutic composition according to any one of claims 1 to 18, wherein the therapeutic composition is a wound dressing.

20. A therapeutic composition according to any preceding claim which is sterile, and packaged in a microorganism-impermeable container.

21. Use of a therapeutic composition according to any one of claims 1 to 18 for the preparation of a medicament for the treatment of a wound.

22. Use according to claim 21, wherein the wound is a chronic wound.

23. Use according to claim 22, wherein the chronic wound is selected from the group consisting of ulcers of venous, arterial or mixed aetiology, decubitus ulcers, or diabetic ulcers.

24. Use of a therapeutic composition according to any one of claims 1 to 18 for the preparation of a medicament for the treatment or prevention of lipodermatosclerosis.

25. A process for the production of a modified polysaccharide for the manufacture of a therapeutic composition according to claim 1, comprising the step of reacting a polysaccharide with a cis-aconitic anhydride.

26. A process according to claim 25, further comprising the step of reacting the polysaccharide with Lawesson's reagent of formula (V):

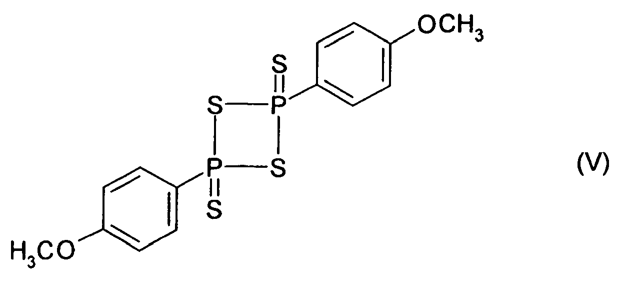

27. A process according to claim 26, wherein said Lawesson's reagent of formula (V) is reacted with said polysaccharide in an amount of at least 0.1 moles per saccharide residue of the polysaccharide.

28. A modified polysaccharide obtainable by a process according to any one of claims 25 to 27.

## Patentansprüche

1. Therapeutische Zusammensetzung, umfassend ein chemisch modifiziertes Polysaccharid, **dadurch gekennzeichnet, daß** auf zumindest einen Saccharidrest eine Seitenkette aufgepfropft worden ist, welche zwei oder mehr Carboxylatgruppen umfaßt, wobei die Seitenkette nur eine Bindung mit dem Polysaccharid aufweist und wobei das modifizierte Polysaccharid hydrophil ist, jedoch eine Löslichkeit von weniger als zirka 1 Gramm pro Liter in Wasser bei 25° C hat.

2. Therapeutische Zusammensetzung nach Anspruch 1, **dadurch gekennzeichnet, daß** ein oder mehr Hydroxylfunktionen auf den Saccharideinheiten oder auf der Seitenkette in eine Thiolfunktion umgewandelt worden sind.

3. Therapeutische Zusammensetzung nach Anspruch 1 oder 2, **dadurch gekennzeichnet, daß** die zwei Carboxylatgruppen auf der Seitenkette 1,1 (geminal), 1,2 (vicinal) oder 1,3 substitutiert worden sind.

4. Therapeutische Zusammensetzung nach Anspruch 2, **dadurch gekennzeichnet, daß** eine oder mehr Hydroxylgruppen auf den Saccharideinheiten in Thiolgruppen umgewandelt worden sind.

5. Therapeutische Zusammensetzung nach Anspruch 2, **dadurch gekennzeichnet, daß** eine oder mehr Hydroxylgruppen auf Seitenketten, die auf die Saccharideinheiten verpflanzt worden sind, in Thiolgruppen umgewandelt worden sind.

6. Therapeutische Zusammensetzung nach einem der vorangehenden Ansprüche, **dadurch gekennzeichnet, daß** die Seitenkette an die Polysaccharidkette durch ein Ester oder eine Amidbindung gebunden ist.

7. Therapeutische Zusammensetzung nach einem der vorangehenden Ansprüche, **dadurch gekennzeichnet, daß** die Seitenkette, welche die Carboxylatgruppen trägt, eine gerade oder verzweigte oder zyklische Gruppe ist, die 1 bis 10 Alkyl- oder Alkenkohlenstoffatome umfaßt, optional unterbrochen durch -O-, -S-, -CO- oder -NH-, optional zumindest eine C-C Doppelbindung enthaltend, und optional weiterhin substituiert mit einem oder mehr Substituenten, ausgewählt aus der Gruppe -OH₂, -NH₂ und Halogen.

8. Therapeutische Zusammensetzung nach einem der vorangehenden Ansprüche, **dadurch gekennzeichnet, daß** zumindest eine der Carboxylatgruppen an ein ungesättigtes Kohlenstoffatom auf der Seitenkette gebunden ist.

9. Therapeutische Zusammensetzung nach einem der vorangehenden Ansprüche, **dadurch gekennzeichnet, daß** das modifizierte Polysaccharid eine Seitenkette von allgemeiner Struktur -X-COCH=C(COOH)CH₂COOH ist, wobei X O oder NH ist.

10. Therapeutische Zusammensetzung nach einem der vorangehenden Ansprüche, **dadurch gekennzeichnet, daß** das modifizierte Polysaccharid modifiziertes Chitin/Chitosan oder eine modifizierte Zellulose oder eine modifizierte Hydroxyalkylzellulose oder ein modifiziertes Alginat ist.

11. Therapeutische Zusammensetzung nach einem der vorangehenden Ansprüche, **dadurch gekennzeichnet, daß** zumindest zirka 1 % der Saccharidreste des modifizierten Polysaccharids modifiziert sind.

12. Therapeutische Zusammensetzung nach Anspruch 11, **dadurch gekennzeichnet, daß** zumindest zirka 5 % der Saccharidreste modifiziert sind.

13. Therapeutische Zusammensetzung nach einem der vorangehenden Ansprüche, **dadurch gekennzeichnet, daß** das modifizierte Polysaccharid eine freie Radikale-Aktivität im Diphenylpikrylhydrazyl (DPPH)-Test auf Antioxidanzien-Aktivität, wie hierin definiert, von zumindest zirka 15 % aufweist.

14. Therapeutische Zusammensetzung nach einem der vorangehenden Ansprüche, die im wesentlichen unlöslich in Wasser ist.

15. Therapeutische Zusammensetzung nach einem der vorangehenden Ansprüche, **dadurch gekennzeichnet, daß** das modifizierte Polysaccharid mit Fe³⁺ Ionen selektiv über Zn²⁺ Ionen komplexbildend ist.

16. Therapeutische Zusammensetzung nach einem der vorangehenden Ansprüche, **dadurch gekennzeichnet, daß** das modifizierte Polysaccharid überwiegend oberflächenmodifiziert ist.

17. Therapeutische Zusammensetzung nach einem der vorangehenden Ansprüche, **dadurch gekennzeichnet, daß** die Carboxylatgruppen Carboxylgruppen sind.

18. Therapeutische Zusammensetzung nach einem der vorangehenden Ansprüche in Form von Perlen, Flocken, Pulver, in Form eines Films, eines faserigen Kissens, eines Gewebes, eines Webstoffs oder eines Faservlieses, eines gefriergetrockneten Schwamms, eines Schaums oder einer Kombination derselben.

19. Therapeutische Zusammensetzung nach einem der Ansprüche 1 bis 18, **dadurch gekennzeichnet, daß** die therapeutische Zusammensetzung ein Wundverband ist.

20. Therapeutische Zusammensetzung nach einem der vorangehenden Ansprüche, welche steril ist und in einem für Mikroorganismen undurchlässigen Behälter verpackt ist.

21. Nutzung einer therapeutischen Zusammensetzung nach einem der Ansprüche 1 bis 18 für die Zubereitung eines Medikaments für die Behandlung einer Wunde.

22. Nutzung nach Anspruch 21, **dadurch gekennzeichnet, daß** die Wunde eine chronische Wunde ist.

23. Nutzung nach Anspruch 22, **dadurch gekennzeichnet, daß** die chronische Wunde ausgewählt wird aus der Gruppe, bestehend aus Geschwüren venöser, arterieller oder gemischter Ätiologie, Dekubitalgeschwüren oder diabetischen Geschwüren.

24. Nutzung einer therapeutischen Zusammensetzung nach einem der Ansprüche 1 bis 18 für die Zubereitung eines Medikaments für die Behandlung oder Verhinderung von Lipodermatosklerose.

25. Verfahren für die Herstellung eines modifizierten Polysaccharids für die Herstellung einer therapeutischen Zusammensetzung nach Anspruch 1, welches den Schritt der Reaktion eines Polysaccharids mit einem Cis-Akonitanhydrid umfaßt.

26. Verfahren nach Anspruch 25, welches weiterhin den Schritt der Reaktion des Polysaccharids mit dem Lawesson's Reagens der Formel (V) umfaßt:

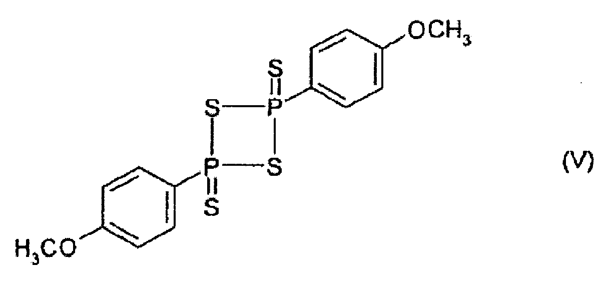

27. Verfahren nach Anspruch 26, **dadurch gekennzeichnet, daß** das Lawesson's Reagens der Formel (V) einer Reaktion mit Polysaccharid in einer Menge von zumindest 0,1 Mol pro Saccharidrest des Polysaccharids ausgesetzt wird.

28. Modifiziertes Polysaccharid, erhältlich durch ein Verfahren nach einem der Ansprüche 25 bis 27.

## Revendications

1. Composition thérapeutique comprenant un polysaccharide chimiquement modifié, dans laquelle au moins un résidu de saccharide s'est greffé sur une chaîne latérale comprenant deux ou plusieurs groupes carboxylates, dans laquelle la chaîne latérale n'a qu'une liaison sur le polysaccharide, et dans laquelle le polysaccharide modifié est hydrophile mais présente une solubilité de moins d'environ 1 gramme par litre dans de l'eau à 25°C.

2. Composition thérapeutique selon la revendication 1, dans laquelle une ou plusieurs fonctions hydroxyles sur les unités de saccharide ou sur la chaîne latérale ont été converties en une fonction thiol.

3. Composition thérapeutique selon la revendication 1 ou 2, dans laquelle les deux groupes carboxylates sont substitués 1,1 (en position géminale), 1,2 (en position vicinale) ou 1,3 sur ladite chaîne latérale.

4. Composition thérapeutique selon la revendication 2, dans laquelle un ou plusieurs groupes hydroxyles sur les unités de saccharide ont été convertis en groupes thiols.

5. Composition thérapeutique selon la revendication 2, dans laquelle un ou plusieurs groupes hydroxyles sur les chaînes latérales greffé(s) sur les unités de saccharide ont été convertis en groupes thiols.

6. Composition thérapeutique selon l'une quelconque des revendications précédentes dans laquelle la chaîne latérale est liée à la chaîne de polysaccharide par une liaison ester ou amide.

7. Composition thérapeutique selon l'une quelconque des revendications précédentes, dans laquelle la chaîne latérale portant les groupes carboxylates est un groupe droit ou ramifié ou cyclique de 1 à 10 atomes de carbone alkyle ou alkylène, facultativement interrompus par -O-, -S-, -CO- ou -NH-, contenant facultativement au moins une double liaison C=C, et en outre substitué facultativement par un ou plusieurs substituants choisis dans le groupe -OH, -NH₂, et halogène.

8. Composition thérapeutique selon l'une quelconque des revendications précédentes, dans laquelle au moins un desdits groupes carboxylates est lié à un atome de carbone insaturé sur ladite chaîne latérale.

9. Composition thérapeutique selon l'une quelconque des revendications précédentes, dans laquelle le polysaccharide modifié comprend une chaîne latérale ayant une structure générale -X-COCH=C(COOH)CH₂COOH, dans laquelle X est O ou NH.

10. Composition thérapeutique selon l'une quelconque des revendications précédentes, dans laquelle le polysaccharide modifié est du chitine/chitosane modifié(e) ou une cellulose modifiée ou une cellulose hydroxyalkyle modifiée ou un alginate modifié.

11. Composition thérapeutique selon l'une quelconque des revendications précédentes, dans laquelle au moins environ 1% des résidus de saccharide dudit polysaccharide modifié est modifié.

12. Composition thérapeutique selon la revendication 11, dans laquelle au moins environ 5% des résidus de saccharide sont modifiés.

13. Composition thérapeutique selon l'une quelconque des revendications précédentes, dans laquelle le polysaccharide modifié a une activité de radical libre dans le test diphénylpicrylhydrazyle (DPPH) relatif à l'activité antioxydante telle que définie dans les présentes d'au moins environ 15%.

14. Composition thérapeutique selon l'une quelconque des revendications précédentes qui est considérablement insoluble dans l'eau.

15. Composition thérapeutique selon l'une quelconque des revendications précédentes, dans laquelle le polysaccharide modifié se complexe avec des ions Fe³⁺ sélectivement sur des ions Zn²⁺.

16. Composition thérapeutique selon l'une quelconque des revendications précédentes, dans laquelle le polysaccharide modifié est d'une manière prédominante modifié en surface.

17. Composition thérapeutique selon l'une quelconque des revendications précédentes, dans laquelle les groupes carboxylates sont des groupes carboxyles.

18. Composition thérapeutique selon l'une quelconque des revendications précédentes sous la forme de perles, de paillettes, de poudre, d'un film, d'un tampon fibreux, d'une toile, d'un tissu tissé ou non tissé, d'une éponge lyophilisée, d'une mousse ou d'une combinaison de ceux-ci.

19. Composition thérapeutique selon l'une quelconque des revendications 1 à 18, dans laquelle la composition thérapeutique est un pansement pour blessures.

20. Composition thérapeutique selon l'une quelconque des revendications précédentes qui est stérile et conditionnée dans un récipient imperméable aux microorganismes.

21. Utilisation d'une composition thérapeutique selon l'une quelconque des revendications 1 à 18 destinée à la préparation d'un médicament destiné au traitement d'une blessure.

22. Utilisation selon la revendication 21, dans laquelle la blessure est une blessure chronique.

23. Utilisation selon la revendication 22, dans laquelle la blessure chronique est choisie dans le groupe constitué par les ulcères d'étiologie veineuse, artérielle ou mixte, les escarres de décubitus ou les ulcères diabétiques.

24. Utilisation d'une composition thérapeutique selon l'une quelconque des revendications 1 à 18 pour la préparation d'un médicament destiné au traitement ou à la prévention de la lipodermatosclérose.

25. Procédé destiné à la production d'un polysaccharide modifié destiné à la fabrication d'une composition thérapeutique selon la revendication 1, comprenant l'étape de la mise en réaction d'un polysaccharide avec un anhydride cis-aconitique.

26. Procédé selon la revendication 25, comprenant en outre l'étape de la mise en réaction du polysaccharide avec un réactif de Lawesson ayant la formule (V):

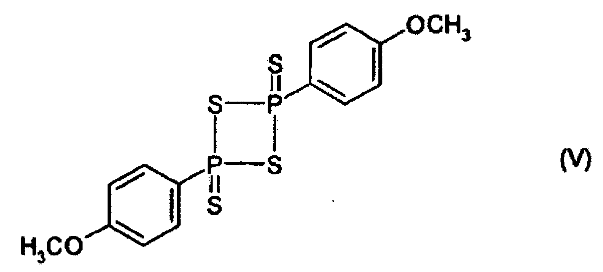

27. Procédé selon la revendication 26, dans laquelle ledit réactif de Lawesson ayant la formule (V) est mis en réaction avec ledit polysaccharide en une quantité d'au moins 0,1 mole par résidu de saccharide du polysaccharide.

28. Polysaccharide modifié pouvant être obtenu par un procédé selon l'une quelconque des revendications 25 à 27.
